(19) Europäisches Patentamt
European Patent Office
Office européen des brevets

(11) **EP 3 590 427 B1**

(12) **EUROPÄISCHE PATENTSCHRIFT**

(45) Veröffentlichungstag und Bekanntmachung des Hinweises auf die Patenterteilung:
**26.08.2020   Patentblatt 2020/35**

(51) Int Cl.:
*A61B 6/00* *(2006.01)*          *A61B 6/12* *(2006.01)*

(21) Anmeldenummer: **18181684.4**

(22) Anmeldetag: **04.07.2018**

(54) **VERFAHREN ZUM ERMITTELN EINER GEEIGNETEN ANGULATION UND VORRICHTUNG**

METHOD FOR DETERMINING A SUITABLE ANGULATION AND DEVICE

PROCÉDÉ DE DÉTERMINATION D'UNE ANGULATION APPROPRIÉE ET DISPOSITIF

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**08.01.2020   Patentblatt 2020/02**

(73) Patentinhaber: **Siemens Healthcare GmbH**
**91052 Erlangen (DE)**

(72) Erfinder: **Bittner, Maik**
**91080 Marloffstein (DE)**

(56) Entgegenhaltungen:
**DE-A1-102010 007 177      DE-A1-102010 022 526**
**DE-A1-102011 006 484**

## Beschreibung

Die Erfindung betrifft ein Verfahren zum Ermitteln einer für eine Visualisierung eines Führungsdrahts geeigneten Angulation eines Aufnahmesystems gemäß dem Patentanspruch 1 sowie ein medizinisches Röntgengerät zur Durchführung eines derartigen Verfahrens gemäß dem Patentanspruch 9.

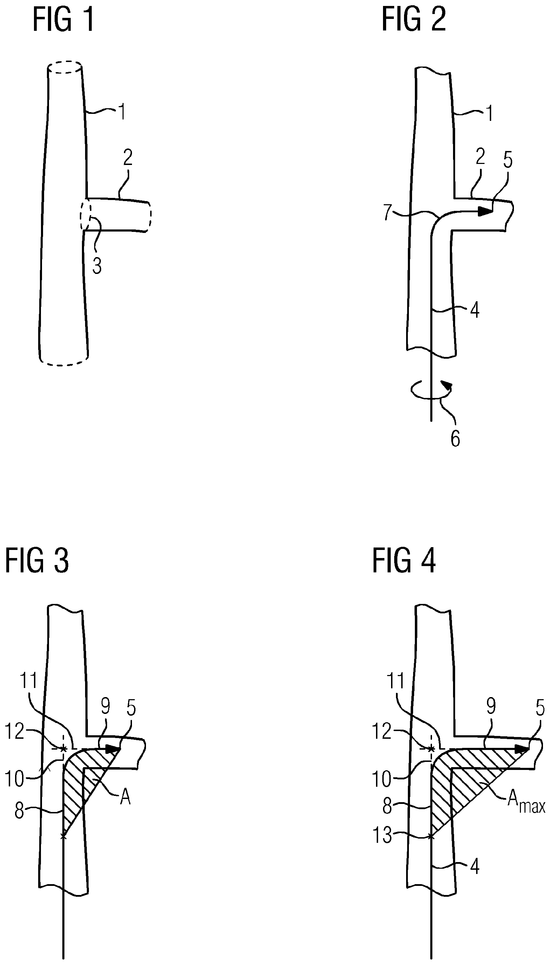

Bei der Durchführung bestimmter endovaskularer Interventionen müssen Führungsdrähte durch Blutgefäße navigiert werden, unter anderem von einem Gefäß 1 in eine Abzweigung des Gefäßes (z.B. in einen Nebenast 2 des Gefäßes)- siehe FIG 1. Dies wird im Allgemeinen unter Echtzeit-Überwachung durch ein Fluoroskopie-Röntgengerät, z.B. ein C-Bogengerät, durchgeführt, wie z.B. aus der DE 10 2011 006 484 A1 bekannt.

Der Führungsdraht 4 wird mittels seiner Spitze 5 gelenkt, wobei durch Drehen des gesamten Führungsdrahtsystems in Drehrichtung 6 die Spitze 5 in Richtung des Zielgefäßes orientiert und dann eingeführt wird - siehe FIG 2.

Um die Prozedur durchzuführen sind im Allgemeinen zwei Ansichten notwendig: (P1) ist die Seitenansicht des Abflusses des Gefäßes 1 in den Nebenast 2; da der Abfluss als virtueller Ring 3 darstellbar ist wird dieser in der Seitenansicht (P1) zu einer Linie, und (P2) ist **die Sicht auf die Ebene,** die der gekrümmte Führungsdraht durch die Krümmung virtuell aufspannt, so dass die Krümmung maximal sichtbar ist. Um diese Ansichten zu ermitteln, muss das Aufnahmesystem des Fluoroskopiegeräts, im Allgemeinen ein C-Bogen, entsprechend unter Gabe von Kontrastmittel so lange gedreht werden, bis die Ansichten dem Benutzer passend erscheinen. Da endovaskulare Untersuchungen bzw. Interventionen hauptsächlich bei multimorbiden und/oder sehr alten Patienten angewendet werden, bei denen ein offener chirurgischer Eingriff ein zu großes Risiko darstellen würde, ist eine möglichst kurze Dauer der Intervention und möglichst geringer Einsatz von Kontrastmittel wünschenswert. Wenn im Röntgenbild keine Krümmung des Führungsdrahtes sichtbar ist, dann ist die Angulation des C-Bogens derart, dass dieser bezüglich seines Zentralstrahls von "hinten" auf die Krümmung schaut. Die Krümmung ist maximal sichtbar, wenn der Zentralstrahl des C-Bogens ein Lot auf die Ebene der Krümmung des Führungsdrahts bildet (P2).

Zur Ermittlung der Seitenansicht (P1) existieren bereits automatische Verfahren. So wird zunächst der Abfluss des Gefäßes in die Abzweigung als virtueller Ring im Fluoroskopie-Röntgenbild dargestellt und das Aufnahmesystem dann so bewegt, dass der virtuelle Ring in der Darstellung zu einer geraden Linie wird (*syngo* EVAR Guidance™ und *syngo* Aortic Valve Guidance™). Zur Ermittlung der Sicht (P2) muss der Benutzer manuell bzw. halbautomatisch den C-Bogen so lange verfahren, bis seiner Ansicht nach die Krümmung maximal sichtbar ist.

Es ist Aufgabe der vorliegenden Erfindung, ein Verfahren bereitzustellen, welches schnell geeignete Ansichten auf einen gekrümmten Führungsdraht von einem Gefäß eines Patienten in eine Abzweigung des Gefäßes ermöglicht; des Weiteren ist es Aufgabe der Erfindung, ein für die Durchführung des Verfahrens geeignetes Röntgengerät bereitzustellen.

Die Aufgabe wird erfindungsgemäß gelöst durch ein Verfahren zum Ermitteln einer für eine Visualisierung eines Führungsdrahts geeigneten Angulation eines Aufnahmesystems gemäß dem Patentanspruch 1 und von einem medizinischen Röntgengerät gemäß dem Patentanspruch 9. Vorteilhafte Ausgestaltungen der Erfindung sind jeweils Gegenstand der zugehörigen Unteransprüche.

Das erfindungsgemäße Verfahren zum automatischen Ermitteln einer für eine Visualisierung eines Führungsdrahts geeigneten Angulation eines Aufnahmesystems während einer Navigation des Führungsdrahts durch ein Gefäßsystem eines Patienten von einem Gefäß in eine Abzweigung des Gefäßes, wodurch der Führungsdraht eine Krümmung und einen ersten, proximalen Abschnitt auf der ersten Seite der Krümmung und einen zweiten, distalen, die Spitze des Führungsdrahtes aufweisenden Abschnitt auf der zweiten Seite der Krümmung aufweist, weist die folgenden Schritte auf:

- Aufnehmen von einer (z.B. schnellen) Abfolge von Live Röntgenbildern des Führungsdrahts und Darstellung der Röntgenbilder an einer Anzeigeeinheit, währenddessen:
- Identifizierung des Führungsdrahts, insbesondere mittels eines Bilderkennungsalgorithmus, in den Röntgenbildern,
- Anlegen einer ersten virtuellen Tangente an den ersten Abschnitt und einer zweiten virtuellen Tangente an den zweiten Abschnitt des Führungsdrahts auf den Röntgenbildern,
- Bestimmung eines Schnittpunktes der virtuellen Tangenten auf den Röntgenbildern,
- Ermittlung eines Dreieckes, dessen drei Eckpunkte der Schnittpunkt und ein erster Punkt auf dem ersten Abschnitt und ein zweiter Punkt auf dem zweiten Abschnitt sind, auf den Röntgenbildern,
- Bestimmung der Fläche des Dreiecks, und
- Ermitteln der geeigneten Angulation des Aufnahmesystems durch Maximierung der Fläche des Dreiecks mittels Bewegung des Aufnahmesystems.

Durch die Erfindung kann automatisch eine optimale Angulation des Aufnahmesystems bezüglich des gekrümmten Führungsdrahts derart, dass der Zentralstrahl des Aufnahmesystems ein Lot auf die durch die Krümmung aufgespannte Ebene bildet, und damit eine optimale Ansicht zur Darstellung der Navigation des Führungsdrahts von einem Gefäß des Patienten in eine Abzweigung des Gefäßes erzielt werden. Hierfür wird die Fläche des virtuellen Dreiecks, dessen Schenkel die virtuellen Tangenten bilden, maximiert bzw. das Aufnahmesystem automatisch so lange bewegt, bis die Fläche des Dreiecks ihr Maximum erreicht.

Der erste Punkt auf dem ersten Abschnitt kann automatisch oder zuvor manuell frei gewählt werden, muss aber - sobald ausgewählt - dann im gesamten Verfahren der gleiche Punkt bleiben. Nach einer Ausgestaltung der Erfindung werden der erste Punkt vom Berührungspunkt der ersten Tangente mit dem ersten Abschnitt und der zweite Punkt vom Berührungspunkt der zweiten Tangente mit dem zweiten Abschnitt gebildet. Unter dem Berührungspunkt wird dabei der Punkt verstanden, an welchem die Tangente erstmalig den jeweiligen Abschnitt berührt. Nach einer weiteren Ausgestaltung der Erfindung wobei der zweite Punkt von der Spitze des Führungsdrahtes gebildet.

Durch das Verfahren kann die optimale Angulation sehr schnell gefunden werden, so dass die Navigation des Führungsdrahtes zügig und damit für den Patienten schonend durchgeführt werden kann. Die Dauer der Röntgenbestrahlung und Dauer der Gabe von Kontrastmittel des Patienten kann durch das Verfahren gesenkt werden, so dass der Patient durch das Verfahren weniger belastet wird als durch Verfahren des Standes der Technik.

Im einfachsten Fall ist das Dreieck rechtwinklig und/oder gleichschenklig.

Nach einer Ausgestaltung der Erfindung wird die geeignete Angulation des Aufnahmesystems ermittelt, indem (a) zuerst das Aufnahmesystem derart positioniert wird, dass der Zentralstrahl ein Lot auf den ersten Abschnitt bildet und (b) danach zur Maximierung der Fläche des Dreiecks das Aufnahmesystem um den ersten Abschnitt bzw. die erste Tangente als Rotationsachse rotiert wird. Es wird also zuerst der erste Abschnitt des Führungsdrahts optimal visualisiert, so dass die abschließende Ausrichtung des Aufnahmesystems lediglich mittels einer Rotation des Aufnahmesystems durchgeführt werden kann. Schritt (a) bezüglich des ersten Abschnitts bzw. der ersten Tangente kann einfach und schnell durchgeführt werden, da das entsprechende Gefäß bzw. der Führungsdraht im Allgemeinen durch Röntgenbilder bereits ausreichend gut dargestellt sind. Daraus ergibt sich dann ein insgesamt sehr schnelles und einfaches Auffinden der geeigneten Angulation.

Alternativ kann auch die geeignete Angulation des Aufnahmesystems ermittelt werden, indem (a) zuerst das Aufnahmesystem derart positioniert wird, dass der Zentralstrahl ein Lot auf den zweiten Abschnitt bildet und (b) danach zur Maximierung der Fläche des Dreiecks das Aufnahmesystem um den zweiten Abschnitt bzw. die zweite Tangente als Rotationsachse rotiert wird.

Nach einer dritten Alternative kann auch die geeignete Angulation des Aufnahmesystems ermittelt werden, indem zur Maximierung der Fläche des Dreiecks das Aufnahmesystem in einer Orbitalbewegung bewegt wird. In diesem Fall wird die Darstellung des ersten und des zweiten Abschnitt sozusagen gleichzeitig optimiert und das Aufnahmesystem bei der Maximierung des virtuellen Dreiecks in mehreren Freiheitsgraden bewegt.

Nach einer weiteren Ausgestaltung der Erfindung werden zur Bestimmung der Fläche des Dreiecks die entsprechenden Pixel auf den Röntgenbildern automatisch gezählt. Alternativ kann auch die Länge der Schenkel des Dreiecks zur Berechnung herangezogen werden.

Nach einer weiteren Ausgestaltung der Erfindung wird die so bestimmte geeignete Angulation zur Visualisierung der Navigation des Führungsdrahts durch ein Gefäßsystem eines Patienten von einem Gefäß in eine Abzweigung des Gefäßes anschließend automatisch eingestellt. Der Behandler kann auf diese Weise die Intervention schnell und unter optimaler Sichtkontrolle durchführen.

Die Erfindung umfasst außerdem ein medizinisches Röntgengerät zur Durchführung des Verfahrens, aufweisend ein in eine Vielzahl von Angulationen bezüglich eines Patienten bewegbares Aufnahmesystem mit einer Röntgenquelle und einem Röntgendetektor zur Aufnahme von Röntgenbildern des Patienten, eine Steuerungseinheit zur Ansteuerung des Röntgengeräts, eine Anzeigeeinheit zur Anzeige der aufgenommenen Röntgenbilder, eine Bildbearbeitungseinheit zur Bearbeitung der Röntgenbilder und eine Berechnungseinheit.

Die Erfindung sowie weitere vorteilhafte Ausgestaltungen gemäß Merkmalen der Unteransprüche werden im Folgenden anhand schematisch dargestellter Ausführungsbeispiele in der Zeichnung näher erläutert, ohne dass dadurch eine Beschränkung der Erfindung auf diese Ausführungsbeispiele erfolgt. Es zeigen:

FIG 1      eine Seitenansicht eines Gefäßes eines Patienten und eine Abzweigung des Gefäßes;

FIG 2      eine Seitenansicht eines Gefäßes und seine Abzweigung mit einem Führungsdraht;

FIG 3      eine Sicht auf einen Führungsdraht mit Dreiecksfläche;

FIG 4      eine geeignete Ansicht auf einen Führungsdraht mit maximaler Dreiecksfläche;

FIG 5      eine Abfolge der Schritte eines erfindungsgemäßen Verfahrens; und

FIG 6      ein erfindungsgemäßes Röntgengerät zur Durchführung des Verfahrens.

Die Figuren 1 und 2 zeigen - wie bereits beschrieben - ein Gefäß 1 eines Patienten und einen Nebenast 2 des Gefäßes, in welchen ein Führungsdraht 4 eingebracht werden soll. Der Führungsdraht weist eine Krümmung 7 auf, welche durch die Navigation in den Nebenast 2 zu Stande kommt. Dies findet häufig im Rahmen von endovaskulären Interventionen statt und zur Überwachung wird mittels medizinischer Fluoroskopie-Röntgengeräte eine Abfolge von Live Röntgenbildern aufgenommen. Um die Prozedur durchzuführen sind im Allgemeinen zwei Ansichten notwendig: (P1) ist die Seitenansicht des Gefäßabflusses des Gefäßes 1 in den Nebenast 2; da der Abfluss als virtueller Ring 3 darstellbar ist wird dieser in der Seitenansicht (P1) zu einer Linie, und (P2) ist die Sicht auf die Ebene, die der gekrümmte Führungsdraht durch die Krümmung virtuell aufspannt, so dass die Krümmung maximal sichtbar ist.

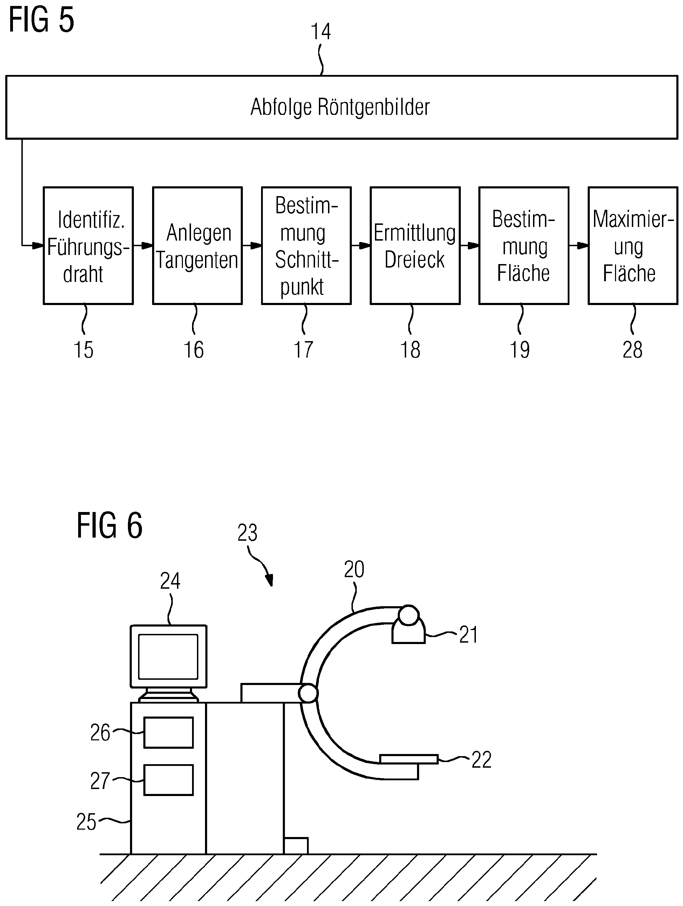

Das erfindungsgemäße Verfahren ermittelt schnell und besonders genau automatisch eine für (P2) geeignete Angulation bzw. Neigung des C-Bogens. In der FIG 5 ist eine Schrittabfolge des erfindungsgemäßen Verfahrens gezeigt. In einem ersten Schritt 14 wird eine Abfolge von Live Röntgenbildern, insbesondere Fluoroskopie Röntgenbildern, des Führungsdrahts aufgenommen und an einer Anzeigeeinheit dargestellt. Die folgenden Schritte zwei bis acht werden während des ersten Schrittes, also während der Echtzeitüberwachung durch Röntgenbilder, durchgeführt.

In einem zweiten Schritt 15 wird auf zumindest einem Teil der Röntgenbilder der Führungsdraht identifiziert. Dies kann z.B. mittels eines Bilderkennungsalgorithmus durchgeführt werden. Solange sich der Führungsdraht und auch das Aufnahmesystem nicht bewegen, kann dann angenommen werden, dass der Führungsdraht auf den Röntgenbildern an derselben Stelle verbleibt. Der identifizierte Führungsdraht kann auf den Röntgenbildern zusätzlich eingeblendet/markiert werden, um die folgenden Schritte zu erleichtern. In den Figuren 3 und 4 ist der Führungsdraht 4 mit einem ersten, proximalen Abschnitt auf der ersten Seite der Krümmung und einem zweiten, distalen, die Spitze 5 des Führungsdrahtes aufweisenden Abschnitt 9 auf der zweiten Seite der Krümmung aufweist.

In einem dritten Schritt 16 wird anschließend eine erste virtuelle Tangente 10 an den ersten Abschnitt und eine zweite virtuelle Tangente 11 an den zweiten Abschnitt des Führungsdrahts angelegt und in einem vierten Schritt 17 der Schnittpunkt 12 der ersten Tangente 10 und der zweiten Tangente 11 bestimmt. Auch diese beiden Schritte können z.B. mittels einer Software und/oder einer Bildbearbeitungseinheit durchgeführt werden.

In einem fünften Schritt 18 wird ein Dreieck ermittelt, dessen drei Eckpunkte der Schnittpunkt und ein erster Punkt 13 auf dem ersten Abschnitt und ein zweiter Punkt auf dem zweiten Abschnitt sind. Grundsätzlich können der erste Punkt auf dem ersten Abschnitt und der zweite Punkt auf dem zweiten Abschnitt beliebig (automatisch oder manuell) gewählt werden, müssen aber in den folgenden Schritten dieselben bleiben. Vorteilhaft für eine einfache Auswahl kann als erster Punkt der (innere) Berührungspunkt der ersten Tangente mit dem ersten Abschnitt und als zweiter Punkt der (innere) Berührungspunkt der zweiten Tangente mit dem zweiten Abschnitt verwendet werden. Alternativ kann z.B. als der zweite Punkt die Spitze des Führungsdrahtes verwendet werden. In einem sechsten Schritt wird die Fläche A des virtuellen Dreiecks bestimmt. Hier können z.B. durch eine Software die entsprechenden Punkte und die Fläche des Dreiecks bestimmt werden. Wenn das Dreieck rechtwinklig ist, dann ist seine Fläche einfach A=ab/2, wobei a und b die Schenkel des Dreiecks sind. Wenn das Dreieck nicht rechtwinklig ist, können die Pixel gezählt werden, um die Fläche zu bestimmen.

Anschließend wird in einem siebten Schritt 28 die Fläche des Dreiecks unter Bewegung des Aufnahmesystems maximiert. Dabei wird das Aufnahmesystem so lange automatisch bewegt, insbesondere gedreht, bis die Fläche des Dreiecks ihr Maximum erreicht. Ist das Maximum erreicht, so kann angenommen werden, dass der Zentralstrahl des Aufnahmesystems ein Lot auf die Ebene der Krümmung des Führungsdrahts bildet und damit die Krümmung maximal sichtbar ist. Die Angulation bzw. Neigung des C-Bogens, bei welcher das Maximum erreicht ist, ist somit optimal geeignet zur Visualisierung der Krümmung.

Die Bewegung des Aufnahmesystems kann automatisch oder durch eine Eingabe getriggert gestartet werden. Es gibt mehrere geeignete Möglichkeiten, das Aufnahmesystem zu bewegen, um das Maximum der Dreiecksfläche zu finden, jeweils ausgehend von der Anfangs-Angulation oder Neigung des Aufnahmesystems. Ist diese beliebig, so kann das Aufnahmesystem in einer Orbitalbewegung bewegt werden.

Alternativ kann zuerst das Aufnahmesystem derart positioniert werden (oder ist bereits zuvor so positioniert worden), dass der Zentralstrahl ein Lot auf den ersten Abschnitt bildet. Dann wird zur Maximierung der Fläche des Dreiecks das Aufnahmesystem um den ersten Abschnitt rotiert.

Als dritte Alternative kann zuerst das Aufnahmesystem derart positioniert werden (oder ist bereits so positioniert), dass der Zentralstrahl ein Lot auf den zweiten Abschnitt bildet, dann wird zur Maximierung der Fläche des Dreiecks das Aufnahmesystem um den zweiten Abschnitt rotiert.

Ist die Angulation oder Neigung des C-Bogens gefunden, bei welcher die Dreiecksfläche ihr Maximum hat, so kann das Aufnahmesystem dann automatisch in diese Angulation oder neigung verfahren werden bzw. dort bleiben, so dass der interventionelle Eingriff fortgeführt werden kann. Ändert sich z.B. die Position des Führungsdrahtes, kann das Verfahren wiederholt werden, um eine neue optimal geeignete Angulation oder Neigung zu finden und einzustellen. Das Verfahren kann derart durchgeführt werden, dass kein Eingriff eines Nutzers notwendig ist. Bei Bedarf kann auch eine Nutzereingabe abgefragt werden, z.B. zur Auswahl der Punkte oder zum Fortführen des Verfahrens.

In der FIG 6 ist ein erfindungsgemäßes medizinisches Röntgengerät 23 gezeigt. Dieses weist z.B. als Aufnahmesystem einen C-Bogen 20 mit einer Röntgenquelle 21 und einem Röntgendetektor 22 auf. Das medizinische Röntgengerät 23 wird von einer Steuereinheit 25 angesteuert und weist eine Anzeigeeiheit 24 zur Anzeige von Röntgenbildern auf. Außerdem ist eine Bildbearbeitungseinheit 26 und eine Berechnungseinheit 27 vorhanden, welche z.B. mittels einer Software die entsprechenden Schritte des Verfahrens durchführen können.

Zur Lösung des Problems (p2) wird an den ersten und den zweiten Abschnitt je eine virtuelle Tangente angelegt. Der zweite Abschnitt sollte immer die Spitze des Führungsdrahtes aufweisen. Die Tangenten schneiden sich oberhalb der Krümmung des Führungsdrahtes. Verbindet man den Schnittpunkt mit den Berührungspunkten der beiden Abschnitte so entsteht ein Dreieck. Das Bildgebungssystem verfolgt den Führungsdraht per Fluoroskopie. Eine Software kann die entsprechenden Punkte und die Fläche des Dreiecks detektieren und bestimmen. Wenn das Dreieck rechtwinklig ist, dann ist seine Fläche $A = \frac{ab}{2}$, wobei a und b die Schenkel des Dreiecks sind. Wenn das Dreieck nicht rechtwinklig ist, können z.B. die Pixel gezählt werden, um die Fläche zu bestimmen. Automatisch oder durch eine Eingabe getriggert bewegt sich das Aufnahmesystem bis die Fläche des Dreiecks maximal ist.

Durch die Erfindung kann automatisch eine optimale Draufsicht des Aufnahmesystems auf die durch den gekrümmten Führungsdraht aufgespannte Ebene derart, dass der Zentralstrahl des Aufnahmesystems ein Lot auf die Ebene bildet, erzielt werden. Dies wird über die Bestimmung der Angulation bzw. Neigung des C-Bogens, bei welcher das vom Führungsdraht aufgespannte Dreieck maximal ist, erreicht. Verglichen mit einer manuellen Einstellung einer derartigen Draufsicht kann dies genauer und schneller erreicht werden. Dies ist vor allem in zeitkritischen und akuten Situationen im OP hilfreich. Zudem kann Dosis gespart und damit der Patient weniger belastet werden. Eine manuelle Einstellung kostet mehr Zeit und ist ungenauer und komplizierter.

Die Verwendung von Markern in der Spitze 5 des Führungsdrahts, an der Krümmung und am ersten Abschnitt des Führungsdrahts kann das Verfahren optimieren, insbesondere in Bezug auf Bilderkennungsalgorithmen und Berechnung der Dreiecksfläche, das Verfahren funktioniert jedoch auch mit allen anderen Führungsdrähten.

Die Erfindung lässt sich in folgender Weise kurz zusammenfassen: Zur Verbesserung der Patientensicherheit bei interventionellen Eingriffen mit Führungsdrähten ist ein Verfahren zum automatischen Ermitteln einer für eine Visualisierung eines Führungsdrahts geeigneten Angulation oder Neigung eines Aufnahmesystems während einer Navigation des Führungsdrahts durch ein Gefäßsystem eines Patienten von einem Gefäß in eine Abzweigung des Gefäßes, wodurch der Führungsdraht eine Krümmung und einen ersten, proximalen Abschnitt auf der ersten Seite der Krümmung und einen zweiten, distalen, die Spitze des Führungsdrahtes aufweisenden Abschnitt auf der zweiten Seite der Krümmung aufweist, mit den folgenden Schritten vorgesehen:

- Aufnehmen von einer Abfolge von Live Röntgenbildern des Führungsdrahts und Darstellung der Röntgenbilder an einer Anzeigeeinheit, währenddessen:
- Identifizierung des Führungsdrahts auf den Röntgenbildern,
- Anlegen einer ersten virtuellen Tangente an den ersten Abschnitt und einer zweiten virtuellen Tangente an den zweiten Abschnitt des Führungsdrahts auf den Röntgenbildern,
- Bestimmung eines Schnittpunktes der virtuellen Tangenten auf den Röntgenbildern,
- Ermittlung eines Dreieckes, dessen drei Eckpunkte der Schnittpunkt und ein erster Punkt auf dem ersten Abschnitt und ein zweiter Punkt auf dem zweiten Abschnitt sind, auf den Röntgenbildern,
- Bestimmung der Fläche des Dreiecks, und
- Ermitteln der geeigneten Angulation oder Neigung des Aufnahmesystems durch Maximierung der Fläche des Dreiecks mittels Bewegung des Aufnahmesystems.

**Patentansprüche**

1. Verfahren zum automatischen Ermitteln einer für eine Visualisierung eines Führungsdrahts geeigneten Angulation oder Neigung eines Aufnahmesystems während einer Navigation des Führungsdrahts durch ein Gefäßsystem eines Patienten von einem Gefäß in eine Abzweigung des Gefäßes, wodurch der Führungsdraht eine Krümmung und einen ersten, proximalen Abschnitt auf der ersten Seite der Krümmung und einen zweiten, distalen, die Spitze des Führungsdrahtes aufweisenden Abschnitt auf der zweiten Seite der Krümmung aufweist, mit den folgenden Schritten:

   • Aufnehmen (14) von einer Abfolge von Live Röntgenbildern des Führungsdrahts und Darstellung der Röntgenbilder an einer Anzeigeeinheit, das Verfahren **dadurch gekennzeichnet** das währenddessen folgende Schritte durchgeführt werden:
   • Identifizierung (15) des Führungsdrahts auf zumindest einem Teil der Röntgenbilder,
   • Anlegen (16) einer ersten virtuellen Tangente an den ersten Abschnitt und einer zweiten virtuellen Tangente an den zweiten Abschnitt des Führungsdrahts,
   • Bestimmung (17) eines Schnittpunktes der virtuellen Tangenten,
   • Ermittlung (18) eines Dreieckes, dessen drei Eckpunkte der Schnittpunkt und ein erster Punkt auf dem ersten Abschnitt und ein zweiter Punkt auf dem zweiten Abschnitt sind,

• Bestimmung (19) der Fläche des Dreiecks, und
• Ermitteln (28) der geeigneten Angulation oder Neigung des Aufnahmesystems durch Maximierung der Fläche des Dreiecks mittels Bewegung des Aufnahmesystems.

2. Verfahren nach Anspruch 1, wobei die geeignete Angulation oder Neigung des Aufnahmesystems ermittelt wird, indem zuerst das Aufnahmesystem derart positioniert wird, dass der Zentralstrahl ein Lot auf den ersten Abschnitt bildet und dass danach zur Maximierung der Fläche des Dreiecks das Aufnahmesystem um den ersten Abschnitt rotiert wird.

3. Verfahren nach Anspruch 1, wobei die geeignete Angulation oder Neigung des Aufnahmesystems ermittelt wird, indem zuerst das Aufnahmesystem derart positioniert wird, dass der Zentralstrahl ein Lot auf den zweiten Abschnitt bildet und dass danach zur Maximierung der Fläche des Dreiecks das Aufnahmesystem um den zweiten Abschnitt rotiert wird.

4. Verfahren nach Anspruch 1, wobei die geeignete Angulation oder Neigung des Aufnahmesystems ermittelt wird, indem zur Maximierung der Fläche des Dreiecks das Aufnahmesystem in einer Orbitalbewegung bewegt wird.

5. Verfahren nach einem der vorangehenden Ansprüche, wobei zur Bestimmung der Fläche des Dreiecks die entsprechenden Pixel auf den Röntgenbildern gezählt werden.

6. Verfahren nach einem der vorangehenden Ansprüche, wobei die geeignete Angulation oder Neigung anschließend eingestellt wird.

7. Verfahren nach einem der vorangehenden Ansprüche, wobei der zweite Punkt von der Spitze des Führungsdrahtes gebildet wird.

8. Verfahren nach einem der vorangehenden Ansprüche, wobei der erste Punkt vom inneren Berührungspunkt der ersten Tangente mit dem ersten Abschnitt und der zweite Punkt vom inneren Berührungspunkt der zweiten Tangente mit dem zweiten Abschnitt gebildet werden.

9. Medizinisches Röntgengerät zur Durchführung eines Verfahrens nach einem der Ansprüche 1 bis 6 während einer Navigation des Führungsdrahts durch ein Gefäßsystem eines Patienten von einem Gefäß in eine Abzweigung des Gefäßes, wodurch der Führungsdraht eine Krümmung und einen ersten, proximalen Abschnitt auf der ersten Seite der Krümmung und einen zweiten, distalen, die Spitze des Führungsdrahtes aufweisenden Abschnitt auf der zweiten Seite der Krümmung aufweist, aufweisend

• ein in eine Vielzahl von Angulationen bezüglich eines Patienten bewegbares Aufnahmesystem mit einer Röntgenquelle und einem Röntgendetektor zur Aufnahme von Röntgenbildern des Patienten,
• eine Steuerungseinheit zur Ansteuerung des Röntgengeräts,
• eine Anzeigeeinheit zur Anzeige der aufgenommenen Röntgenbilder,
• eine Bildbearbeitungseinheit zur Bearbeitung der Röntgenbilder und eine Berechnungseinheit,

wobei die medizinische Röntgengerät dazu ausgebildet ist, automatisch einer erste virtuellen Tangente an den ersten Abschnitt und einer zweiten virtuellen Tangente an den zweiten Abschnitt anhand der dargestellten Röntgenbilder anzulegen, einen Schnittpunkt der virtuellen Tangenten anhand der dargestellten Röntgenbilder zu bestimmen, ein Dreieck, dessen drei Eckpunkte der Schnittpunkt, die Spitze des Führungsdrahtes und ein willkürlich festgelegter Punkt auf dem ersten Abschnitt sind, anhand der dargestellten Röntgenbilder zu ermitteln, die Fläche des Dreiecks anhand der dargestellten Röntgenbilder zu bestimmen, und eine geeignete Angulation oder Neigung des Aufnahmesystems durch Maximierung der Fläche des Dreiecks mittels Bewegung des Aufnahmesystems zu ermitteln.

**Claims**

1. Method for automatically determining a suitable angulation or inclination of a recording system for a visualisation of a guide wire during a navigation of the guide wire through a vascular system of a patient from a vessel into a branch of the vessel, whereby the guide wire has a curvature and a first proximal section on the first side of the curvature and a second distal section having the tip of the guide wire on the second side of the curvature, with the following steps:

• Recording (14) of a sequence of live X-ray images of the guide wire and presentation of the X-ray images on a display unit, the method **characterised in that** during this process the following steps are carried out:
• Identifying (15) the guide wire on at least some of the X-ray images,
• Applying (16) a first virtual tangent to the first section and a second virtual tangent to the second section of the guide wire,
• Determining (17) a point of intersection of the virtual tangents,

• Determining (18) a triangle, the three corners of which are the point of intersection and a first point on the first section and a second point on the second section,
• Determining (19) the area of the triangle, and
• Determining (28) the suitable angulation or inclination of the recording system by maximising the area of the triangle by means of movement of the recording system.

2. Method according to claim 1, wherein the suitable angulation or inclination of the recording system is determined by first positioning the recording system in such a way that the central beam forms a perpendicular on the first section and that thereafter the recording system is rotated around the first section to maximise the area of the triangle.

3. Method according to claim 1, wherein the suitable angulation or inclination of the recording system is determined by first positioning the recording system in such a way that the central beam forms a perpendicular on the second section and that thereafter the recording system is rotated around the second section to maximise the area of the triangle.

4. Method according to claim 1, wherein the suitable angulation or inclination of the recording system is determined by means of movement of the recording system in an orbital motion to maximise the area of the triangle.

5. Method according to one of the preceding claims, wherein the corresponding pixels on the X-ray images are counted to determine the area of the triangle.

6. Method according to one of the preceding claims, wherein the suitable angulation or inclination is subsequently adjusted.

7. Method according to one of the preceding claims, wherein the second point is formed by the tip of the guide wire.

8. Method according to one of the preceding claims, wherein the first point is formed by the internal point of contact of the first tangent with the first section and the second point by the internal point of contact of the second tangent with the second section.

9. Medical X-ray device for carrying out a method according to one of claims 1 to 6 during a navigation of the guide wire through a vascular system of a patient from a vessel into a branch of the vessel, whereby the guide wire has a curvature and a first proximal section on the first side of the curvature and a second distal section having the tip of the guide wire on the second side of the curvature, having

• A recording system which can be moved in a multiplicity of angulations with regard to a patient, with an X-ray source and an X-ray detector for recording X-ray images of the patient,
• A control unit for controlling the X-ray device,
• A display unit for displaying the recorded X-ray images,
• An image processing unit for processing the X-ray images and a calculation unit,

wherein the medical X-ray device is designed to automatically apply a first virtual tangent to the first section and a second virtual tangent to the second section based on the X-ray images shown, to determine a point of intersection of the virtual tangents based on the X-ray images shown, to determine a triangle, the three corners of which are the point of intersection, the tip of the guide wire and an arbitrarily determined point on the first section, based on the X-ray images shown, to determine the area of the triangle based on the X-ray images shown, and to determine a suitable angulation or inclination of the recording system by maximising the area of the triangle by means of movement of the recording system.

**Revendications**

1. Procédé de détermination automatique d'une angulation ou d'une inclinaison, appropriée à la visualisation d'un fil de guidage, d'un système d'enregistrement pendant une navigation du fil de guidage dans un système de vaisseau d'un patient, d'un vaisseau à une bifurcation du vaisseau, le fil de guidage ayant une courbure et un premier tronçon proximal, du premier côté de la courbure, et, du deuxième côté de la courbure, un deuxième tronçon distal ayant la pointe du fil de guidage, comprenant les stades suivants :

• enregistrement (14) d'une suite d'images aux rayons X Live du fil de guidage et représentation des images aux rayons X sur une unité d'affichage, le procédé étant **caractérisé en ce que** l'on effectue pendant cela les stades suivants :
• identification (15) du fil de guidage sur au moins une partie des images aux rayons X,
• tracé (16) d'une première tangente virtuelle sur le premier tronçon et d'une deuxième tangente virtuelle sur le deuxième tronçon du fil de guidage,
• détermination (17) d'un point d'intersection de tangentes virtuelles,
• construction (18) d'un triangle, dont les trois sommets sont le point d'intersection et un premier point sur le premier tronçon et un deuxième point sur le deuxième tronçon,
• détermination (19) de la surface du triangle, et
• établissement (28) de l'angulation ou de l'inclinaison appropriée du système d'enregistrement en maximisant la surface du triangle en déplaçant le système d'enregistrement.

**2.** Procédé suivant la revendication 1, dans lequel on établit l'angulation ou l'inclinaison appropriée du système d'enregistrement en mettant d'abord en position le système d'enregistrement, de manière à ce que le rayon central soit perpendiculaire au premier tronçon et à ce qu'ensuite, pour maximiser la surface du triangle, on fasse tourner le système d'enregistrement autour du premier tronçon.

**3.** Procédé suivant la revendication 1, dans lequel on établit l'angulation ou l'inclinaison appropriée du système d'enregistrement en mettant d'abord en position le système d'enregistrement, de manière à ce que le rayon central soit perpendiculaire au deuxième tronçon et en faisant tourner ensuite le système d'enregistrement autour du deuxième tronçon pour maximiser la surface du triangle.

**4.** Procédé suivant la revendication 1, dans lequel on établit l'angulation ou l'inclinaison appropriée du système d'enregistrement en déplaçant le système d'enregistrement en un mouvement orbital pour maximiser la surface du triangle.

**5.** Procédé suivant l'une des revendications précédentes, dans lequel, pour déterminer la surface du triangle, on compte les pixels correspondants sur les images aux rayons X.

**6.** Procédé suivant l'une des revendications précédentes, dans lequel on règle ensuite l'angulation ou l'inclinaison appropriée.

**7.** Procédé suivant l'une des revendications précédentes, dans lequel le deuxième point est formé par la pointe du fil de guidage.

**8.** Procédé suivant l'une des revendications précédentes, dans lequel on forme le premier point par le point de contact intérieur de la première tangente avec le premier tronçon et le deuxième point par le point de contact intérieur de la deuxième tangente avec le deuxième tronçon.

**9.** Appareil de rayons X médical pour effectuer un procédé suivant l'une des revendications 1 à 6 pendant une navigation du fil de guidage dans un système de vaisseau d'un patient, d'un vaisseau à une bifurcation du vaisseau, grâce à quoi le fil de guidage a une courbure et un premier tronçon proximal du premier côté de la courbure et du deuxième côté de la courbure un deuxième tronçon distal comportant la pointe du fil de guidage, comportant

• un système d'enregistrement pouvant se déplacer dans une pluralité d'angulations par rapport à un patient et ayant une source de rayons X et un détecteur de rayons X pour l'enregistrement d'images aux rayons X du patient,
• une unité de commande pour commander l'appareil aux rayons X,
• une unité d'affichage pour afficher les images aux rayons X enregistrées,
• une unité de traitement d'images pour traiter les images aux rayons X et une unité de calcul,

dans lequel l'appareil de rayons X médical est constitué pour tracer automatiquement une première tangente virtuelle sur le premier tronçon et une deuxième tangente virtuelle sur le deuxième tronçon à l'aide des images aux rayons X représentées pour déterminer un point d'intersection des tangentes virtuelles à l'aide des images aux rayons X représentées pour construire un triangle dont les trois sommets sont le point d'intersection, la pointe du fil de guidage et un point fixé habituellement sur le premier tronçon pour déterminer la surface du triangle à l'aide des images aux rayons X représentées et par établir une angulation ou une inclinaison appropriée du système d'enregistrement par la maximisation de la surface du triangle en déplaçant le système d'enregistrement.

# FIG 1

# FIG 2

# FIG 3

# FIG 4

# FIG 5

14

| Abfolge Röntgenbilder |
|---|

| Identifiz. Führungs-draht | Anlegen Tangenten | Bestim-mung Schnitt-punkt | Ermittlung Dreieck | Bestim-mung Fläche | Maximier-ung Fläche |
|---|---|---|---|---|---|

15      16      17      18      19      28

# FIG 6

23

24

20

21

26

27

25

22

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102011006484 A1